# EUROPÄISCHE PATENTSCHRIFT

(11) **EP 3 058 022 B1**
(45) Veröffentlichungstag und Bekanntmachung des Hinweises auf die Patenterteilung: **14.06.2017**
(21) Anmeldenummer: 14838769.9
(22) Anmeldetag: 14.11.2014
(51) Int. Cl.: C08L 1/00, C08J 3/12, C08B 15/08, C08L 1/02

(54) **DREIDIMENSIONALER CELLULOSISCHER FORMKÖRPER, VERFAHREN ZU SEINER HERSTELLUNG UND SEINE VERWENDUNG**
THREE-DIMENSIONAL CELLULOSE MOULDED BODY, METHOD FOR THE PRODUCTION THEREOF AND USE OF THE SAME
CORPS MOULÉ CELLULOSIQUE TRIDIMENSIONNEL, ET PROCÉDÉ DE PRODUCTION ET UTILISATION DUDIT CORPS MOULÉ

(43) Veröffentlichungstag der Anmeldung: 24.08.2016
(73) Patentinhaber: Lenzing AG, 4860 Lenzing (AT)
(72) Erfinder: HÄUBL, Martin, A-4040 Linz (AT); INNERLOHINGER, Josef, A-4880 Berg im Attergau (AT); SCHIRK, Christian, A-4810 Gmunden (AT)
(74) Vertreter: Hanemann, Otto
(86) Internationale Anmeldenummer: PCT/AT2014/000202
(87) Internationale Veröffentlichungsnummer: WO 2015/054711

(56) Entgegenhaltungen:
- WO-A1-2009/036480
- WO-A1-2009/065891
- AT-B- 401 779
- M. Abu-Rous: "VISUALISATION OF THE NANO-STRUCTURE OF TENCEL® (LYOCELL)AND OTHER CELLULOSICS AS AN APPROACH TO EXPLAININGFUNCTIONAL AND WELLNESS PROPERTIES IN TEXTILES", , 25. Juli 2006 (2006-07-25), XP002739480, Gefunden im Internet: URL:http://www.lenzing.com/fileadmin/templ ate/pdf/konzern/lenzinger_berichte/ausgabe _85_2006/LB_2006_AbuRous_06_ev.pdf [gefunden am 2015-05-12]
- Martin Gericke: "Functional Cellulose Beads: Preparation, Characterization, and Applications", , 29. März 2013 (2013-03-29), XP002739481, Gefunden im Internet: URL:http://pubs.acs.org/doi/ipdf/10.1021/c r300242j [gefunden am 2015-05-12]
- TRYGG J. ET AL.: "Physicochemical design of the morphology and ultrastructure of cellulose beads", CARBOHYDRATE POLYMERS, Bd. 93, 2013, Seiten 291-299, XP002739482,

## Beschreibung

Die vorliegende Erfindung beschreibt einen neuartigen Typ von Cellulose-II-Partikeln sowie geeignete Herstellungsverfahren. Die Eigenschaften dieser Partikel machen sie speziell zum Einsatz in Kosmetik- und Pharmaanwendungen geeignet. Die Partikel zeichnen sich durch eine schwammartige Feinstruktur im Inneren, welche von einer kompakten Außenhülle umgeben ist, aus.

### Stand der Technik:

Cellulosepulver und andere Systeme, die partikuläre Cellulose enthalten, sind bereits seit Langem bekannt, wobei es auch auf diesem Gebiet speziell in den letzten Jahren vermehrt neue Entwicklungen gegeben hat. Am weitesten verbreitet sind trockene faserige Pulver, die durch das Zerkleinern von Zellstoff mittels geeigneten Aggregaten entstehen. Je nach eingesetztem Zellstoff und Art der Aufbereitung (Zerkleinerung samt allfälligen Modifikationen) lassen sich unterschiedlichste Qualitäten erzeugen. Als eine Variante kann hier auch angesehen werden, dass nicht Zellstoff, sondern direkt Pflanzen(teile) zerkleinert werden. Die erhaltenen Partikel enthalten neben Cellulose dann allerdings auch noch andere Substanzen wie Lignin oder Hemicellulosen in höheren Anteilen und weisen größere Schwankungen hinsichtlich Homogenität auf. Aufgrund der makromolekularen Struktur der Cellulose sind alle bisher beschriebenen Pulver faserig, d. h. die Partikel weisen ein ausgeprägtes L/D-Verhältnis auf.

Eine weitere weit verbreitete Klasse von Cellulosepulvern sind die sogenannten mikrokristallinen Cellulosen (MCC). Bei der Herstellung von MCC aus Zellstoff erfolgt zusätzlich zur mechanischen Zerkleinerung auch eine Behandlung mit Säure, wodurch die amorphen Anteile der Cellulose abgebaut werden und ein Material mit hohem kristallinem Anteil entsteht. Je nach Verfahrensführung können die einzelnen Kristallite in unterschiedliche Formen gebracht werden. So sind hier neben faserigen Partikeln auch Aggregate/Agglomerate von annähernd sphärischer Form möglich. Bei MCC oder konventionellem faserigem Cellulosepulver sind die Möglichkeiten, um zusätzliche Materialien in die Partikel zu inkorporieren oder aufzubringen, beschränkt. Eine Applikation von Additiven ist nur als Beschichtung oder als Einlagerung in den Aggregaten bzw. Agglomeraten möglich. Ein häufig eingesetztes Verfahren ist dabei die Granulation, wobei die gewünschten finalen Partikel aus kleineren Partikeln aufgebaut werden. Teilweise sind diese Ausgangspartikel selbst zuvor wieder durch Mahlung hergestellt worden, was den gesamten Prozess aufwändig macht.

Alle bisher beschriebenen Materialien weisen die gleiche Mikrostruktur, also eine Anordnung der Cellulosemoleküle auf, die in der Fachliteratur als Strukturtyp bezeichnet wird - in diesem Fall die Cellulose-I Struktur. Dieser Typ ist jener, der von Pflanzen gebildet wird und durch die Verfahren, die bei der Herstellung der bereits beschriebenen Partikel eingesetzt werden, auch nicht verändert wird.

Neben Cellulose-I existiert noch ein weiterer häufiger Strukturtyp, der als Cellulose-II bezeichnet wird und die thermodynamisch stabilere Form darstellt. Die beiden Strukturtypen lassen sich röntgenografisch oder auch per NMR einfach unterscheiden. Cellulose-I kann durch Lösen in geeigneten Lösungsmitteln und nachfolgendes Regenerieren in Cellulose-II übergeführt werden. Dieser Prozess findet auch in industriell eingesetzten Verfahren zur Faserproduktion Anwendung, wie unter anderem im Viskose- und im Lyocell-Verfahren. In der letzten Zeit gibt es auch eine Vielzahl von Publikationen zum Thema "Ionische Flüssigkeiten als Lösemittel für Cellulose", wobei hier die großtechnische Umsetzung noch aussteht. Ein Vorteil all dieser Verfahren ist, dass durch das Lösen und darauffolgende Regenerieren der Cellulose eine deutlich variablere Formgebung der Partikel möglich ist. So sind hier auch tatsächlich nativ sphärische Partikel möglich, ohne dass diese wie bei MCC aus Untereinheiten aufgebaut werden müssen. Ein weiterer Vorteil besteht darin, dass im Laufe der Verfahren auch zusätzliche Substanzen direkt in die Partikel inkorporiert und selbstverständlich auch oberflächlich aufgebracht werden können.

Neben diesen trockenen Pulvern aus faserigen oder sphärischen Partikeln gibt es auch noch Suspensionen von cellulosischen Partikeln, oft auch als Cellulosegele bezeichnet. Die einfachste Methode zu deren Herstellung besteht darin, ein geeignetes Cellulosepulver in Wasser zu dispergieren. Allerdings geht der Trend doch deutlich zu ausgeklügelteren Verfahren und Materialien. In letzter Zeit stehen vor allem mikro- und nanoskalierte Cellulosesuspensionen im Zentrum des Interesses. Sie kommen unter unterschiedlichsten Bezeichnungen wie etwa Mikrofibrillierte Cellulose (MFC) oder Nanocellulose vor. Solche Materialien können nun wieder entweder auf Basis von Cellulose-I oder Cellulose-II hergestellt werden. Die Verfahren zur Herstellung sind hier meist sehr aufwändig und energieintensiv sowie nur bedingt auf größere Maßstäbe übertragbar.

Wie dieser grobe Überblick bereits zeigt, ist dem Fachmann eine Vielzahl an cellulosischen Partikelsystemen bekannt, aus denen er auswählen kann. So sind auch die Anwendungsgebiete für cellulosische Partikel inzwischen mannigfaltig und reichen vom Baubereich über Kunststoffverstärkung bis hin zu Pharmazie und Kosmetik. Mit den bekannten Partikeln lassen sich viele Anwendungen abdecken, was aber zum Teil mit zusätzlichem Aufwand verbunden ist, da die Anforderungen von den vorhandenen Materialien nicht komplett erfüllt werden und zusätzliche Verarbeitungsschritte (Modifikationen) notwendig sind.

Neben reiner Cellulose kommen (speziell auch in der Kosmetik) etliche Cellulosederivate (wie z. B. Methylcellulose oder Carboxymethylcellulose) zum Einsatz, die zum Teil wasserlöslich sind. Zusätzlich gibt es eine Vielzahl von Partikeln auf mineralischer oder synthetischer Ausgangsbasis, wobei speziell bei letzteren die Modifikationsmöglichkeiten deutlich vielfältiger sind. Gerade aufwändige kosmetische Produkte enthalten eine Vielzahl von Inhaltsstoffen, um die gewünschten Effekte zu erzielen, was natürlich auch die Formulierung zu einem komplexen Vorgang macht.

US 2010/0297445 beschreibt die Herstellung von Kugeln aus Polymeren, unter anderem cellulosische Kugeln aus einer Celluloselösung in sogenannten organischen Lösungsmitteln wie 1-Ethyl-3-Methyl-Imidazoliumacetat unter Verwendung eines Unterwassergranulators. Die Kugeln werden anschließend über einen Lösungsmittelaustausch getrocknet. Über die innere Struktur dieser Kugeln ist nichts offenbart. Auch die Aufarbeitung der Kugeln nach der Formgebung, wie etwa Waschen oder das Entfernen von Restlösemittel, ist nicht näher spezifiziert.

Die WO 2009/036480 A1 offenbart die Herstellung sphärischer Cellulosepartikel aus einer möglichst amorphen Celluloselösung, wobei mehrere Zerkleinerungsschritte stattfinden. Dieses Dokument offenbart, dass in der ersten Zerkleinerungsstufe neben anderen Aggregaten auch ein Unterwassergranulator eingesetzt werden kann. Welche Struktur das so hergestellte Zwischenprodukt hat, wird nicht offenbart.

Anschließend wird das so hergestellte Zwischenprodukt im nie getrockneten Zustand weiter zerkleinert.

In WO 2009/037146 werden ebenfalls Celluloseperlen offenbart. Diese sind durch verschiedene Verfahren quervernetzt, um die Festigkeit zu erhöhen. Die Quervernetzung bewirkt auch einen fast vollständigen Verlust der Quellfähigkeit der Celluloseperlen.

Die WO 02/57319 offenbart monodisperse Cellulosekugeln, macht aber keinerlei quantitative Aussagen über deren innere Struktur. Diese Kugeln werden aus Lösungen mit maximal 10 Gew.-% Cellulose hergestellt; in den Beispielen wird jedoch mit maximal 4 Gew.-% Cellulose gearbeitet, was darauf schließen läßt, dass die Verwendung höher konzentrierter Celluloselösungen mit der Erfindung gemäß der WO 02/57319 nicht möglich war.

US 2004/011690 beschreibt die Herstellung von Celluloseperlen für den Einsatz in Kosmetik, Pharmazie oder ähnlichen Bereichen. Die Celluloseperlen werden durch Agglomeration von mikrokristalliner Cellulose mit zusätzlichen Additiven gebildet. Die Celluloseperlen sind daher eher ein Mikrogranulat- bzw. Agglomerat und keine kompakten Cellulosepartikel. Die Struktur der Celluloseperlen wird nicht im Detail offengelegt

Ein Review über Celluloseperlen ist zu finden in "M. Gericke et. A., Functional Cellulose Beads: Preparation, Characterization and Applications. Chemical Reviews 113, 2013, S. 4512-4836". Die beschriebenen Herstellungsmethoden beschränken sich jedoch großteils auf Labormethoden. Unterschiedliche Morphologien werden zwar genannt, jedoch nicht im Detail beschrieben, und die Zusammenhänge zwischen Herstellung und Struktur werden nur angedeutet. Der Grossteil des Artikels beschäftigt sich mit der weiteren Funktionalisierung von Celluloseperlen.

Eine detailliertere Beschreibung der Zusammenhänge zwischen der Herstellung von Celluloseperlen und deren Struktur ist zu finden in "J. Trygg et. al., Physicochemical design of the morphology and ultrastructure of cellulose beads. Carbohydrate Polymers 93, 2013, p.291-299." Beschrieben wird das Lösemittelsystem NaOH-Harnstoff mit Cellulosekonzentrationen bis 6%. Unter bestimmten Bedingungen können gemäß diesem Dokument Kern-Hülle-Strukturen erzeugt werden, wobei die Hülle allerdings meist nur wenige µm dick ist. Nur unter extremen Regenerationsbedingungen (10-molare Salpetersäure) kann die Dicke der Haut auf 50 µm gesteigert werden; ansonsten lässt sie sich nur wenig beeinflussen. Eine genauere Charakterisierung der Hülle (beispielsweise im Vergleich zum Kern) findet nicht statt.

Zusammenfassend muss also festgestellt werden, dass im Stand der Technik für Anwendungen in Kosmetik und Pharmazie nur solche cellulosische Partikel mit funktionalen Eigenschaften wie langsame, kontrollierte Freigabe ("Slow-Release") von pharmazeutischen oder kosmetischen Wirkstoffen, Veränderung der Eigenschaften durch äußere Einwirkung, etwa auf Druck ("Stimuli-Response") und ("Sensoric-Booster")-Eigenschaften in Öl/Wasser-Emulsionen bekannt waren, die durch Agglomeration noch kleinerer Partikel hergestellt worden waren. Ein solches vielstufiges Verfahren ist entsprechend aufwendig und damit teuer. Unter anderem ist der Mahlschritt zum Herstellen der Subpartikel sehr energieintensiv. Andererseits sind im Stand der Technik zwar auch bereits cellulosische Partikel bekannt, die die für die obigen Anwendungen erforderliche Partikelgröße bereits aufweisen, aber diese Partikel weisen nicht die oben genannten funktionalen Eigenschaften auf.

### Problemstellung

Angesichts dieses Standes der Technik besteht speziell in den höherwertigen Anwendungsbereichen Kosmetik und Pharmazie immer noch ein Bedarf nach Cellulosepartikeln mit entsprechend verbesserten Eigenschaften. Die Partikel sollten diese Eigenschaften bereits von sich aus mitbringen und diese nicht erst durch einen zusätzlichen Schritt in der Herstellung oder durch Zugabe von weiteren Hilfsmitteln erhalten, wie bis jetzt zum Teil nötig. Folgende Charakteristika sind hierbei von besonderem Interesse: Langsame, kontrollierte Freigabe - "Slow-Release" - von Wirkstoffen (pharmazeutisch oder kosmetisch), Veränderung der Eigenschaften durch äußere Einwirkung - "Stimuli-Response" - (etwa auf Druck), verbesserte Quellbarkeit, definierte innere Struktur (beispielsweise eine klar definierte, reproduzierbar herstellbare Kern-Mantel-Struktur) und definierte Oberflächenbeschaffenheit. Diese und gegebenenfalls weitere Funktionalitäten sollen während der Herstellung der Partikel definiert eingebracht werden. Zusätzlich sollen die Herstellungsverfahren möglichst einfach und somit großtechnisch gut realisierbar sein. Durch die Multifunktionalität dieser Partikel soll im Idealfall auch die Formulierung und Herstellung der Endprodukte vereinfacht werden, da insgesamt dann weniger Inhaltsstoffe notwendig sind. Ein weiterer Vorteil könnte sich ergeben, wenn durch solche neuartigen Cellulose-Partikel auch synthetische Materialien ersetzt werden können. Dadurch könnte der verstärkten Nachfrage nach Produkten aus nachwachsenden Rohstoffen Rechnung getragen werden.

Die erfindungsgemäße Lösung der oben beschriebenen Aufgabe bestand nun darin, die Cellulosepartikel nicht aus Subpartikeln aufzubauen, sondern sie in einem Schritt herzustellen. Besonderes Augenmerk gilt dabei der Wahl der entsprechenden Verfahrensparameter, da durch diese bereits die Eigenschaften der Partikel definiert werden.

### Beschreibung der Erfindung

Diese Aufgabe konnte nun gelöst werden durch einen dreidimensionalen cellulosischen Formkörper gemäss Anspruch 1, der eine optisch feststellbare Kern-Mantel-Struktur aufweist, wobei der Mantel eine höhere Dichte und eine niedrigere Kristallinität als der Kern aufweist und der Kern eine schwammähnliche Struktur aufweist. "Optisch feststellbar" bedeutet im Zusammenhang mit dieser Erfindung, daß die Kern-Mantel-Struktur mit Hilfe der Lichtmikroskopie, Röntgen- und/oder NMR-Spektroskopie festgestellt werden kann. Dabei eignet sich die die Mikroskopie sowohl für trockene als auch gequollene Proben. Röntgen ist hingegen auf trockene, insbesondere lufttrockene, Proben und NMR-Spektroskopie auf Formkörper im gequollenen Zustand beschränkt. Dies ist auf die Notwendigkeit der Probenpräparation zurückzuführen. Da die erfindungsgemäßen Formkörper aus einer Celluloselösung hergestellt werden, weisen sie immer den Strukturtyp Cellulase-II auf.

Der Mantel des erfindungsgemäßen Formkörpers weist eine relative Dichte von 65% bis 85% und der Kern eine relative Dichte von 20% bis 60% auf. Die relative Dichte ist dabei auf kompakte Cellulose bezogen. Die Manteldicke beträgt zwischen 50 µm und 200 µm.

Das Verhältnis von Manteldicke zu Gesamtdurchmesser des Formkörpers beträgt zwischen 1:5 und 1:50. Die erfindungsgemäßen Formkörper sind bevorzugt im Wesentlichen sphärisch, können aber auch zylindrisch, ellipsoid oder ovoid sein. Das Verhältnis der Halbachsen (Länge : Durchmesser) des Formkörpers übersteigt 3:1 nicht. Die erfindungsgemäßen Formkörper können je nach Anwendungszweck entweder getrocknet oder im nie getrocknetem Zustand weiterverwendet werden, wobei die nie getrocknete Variante der Celluloseperlen bevorzugt einen Feuchtegehalt von 25 - 300 Gew.-%, bezogen auf die Cellulosemenge, aufweist.

Die erfindungsgemäßen Formkörper können je nach Anwendungszweck Additivsubstanzen enthalten, die während ihrer Herstellung inkorporiert wurden. Diese Additivsubstanzen sind bevorzugt ausgewählt aus der Gruppe enthaltend ZnO, TiO₂, CaCO₃, CaCl₂, Kaolin, Fe₂O₃, Aluminiumhydroxid, Kaolin,.Farbpigmente auf Kunststoffbasis, Aktivkohle, Superabsorbermaterialien, Phase-change-Materialien, Flammschutzmittel, Biozide, Chitosan sowie weitere Polymere und Biopolymere.

Weiterhin weisen die erfindungsgemäßen Formkörper ein hohes Wasserrückhaltevermogen (WRV) auf. Das WRV liegt beispielsweise nach 2 h Quellung in VE-Wasser für bei Normaldruck getrockneten Celluloseperlen typischerweise im Bereich von 70 - 90 Gew.-% und bei superkritisch CO₂-getrockneten oder gefriergetrockneten Celluloseperlen typischerweise im Bereich von 120 - 150 Gew.-%.

Gegenstand der vorliegenden Erfindung ist weiterhin ein Verfahren zur Herstellung des oben beschriebenen, erfindungsgemäßen dreidimensionalen cellulosischen Formkörpers mit optisch feststellbarer Kern-Mantel-Struktur, dadurch gekennzeichnet, dass es folgende Herstellungsschritte umfasst:
a. Auflösung der Cellulose gemäß einem Lyocellprozess, um eine Lösung mit 10 bis 15 Gew.-% Cellulose zu erhalten;
b. Extrusion der in Schritt a. erhaltenen Celluloselösung ohne Luftspalt direkt in ein Fällbad;
c. Regenerationsprozess, wobei beim Eintritt der Celluloselösung ins Fällbad die Differenz der NMMO-Konzentrationen von Celluloselösung und Fällbad 15 - 78 Gew.-%, bevorzugt 40 - 70 Gew.-%, und die Differenz der Temperatur von Celluloselösung und Fällbad 50 - 120 K, bevorzugt 70 - 120 K, besonders bevorzugt 80 - 120 K betragen sollen;
d. Waschprozess gemäß dem Perkolationsprinzip mit mindestens einem alkalischen Waschschritt, bevorzugt bei pH 9-13;
e. optional einem Trocknungsprozess, durch den die Außenhaut der Formkörper nicht abrasiv verletzt wird;
wobei die in Punkt d.) genannte Wäsche bevorzugt mehrstufig und im Gegenstrom erfolgt und mindestens einen alkalischen Schritt enthält.

Als Löseprozesse kommen etwa das Viskose-, das Lyocell- oder das Cuprammonium-Verfahren in Betracht; auch Lösen der Cellulose in NaOH oder geeigneten ionischen Flüssigkeiten ist möglich. Generell ist die Erfindung nicht auf bestimmte Lösemittel bzw. Prozesse eingeschränkt, sondern durch den Einsatz unterschiedlicher Verfahren kann die Struktur der erhaltenen Partikel zusätzlich beeinflusst werden. Bevorzugt ist jedoch das dem Fachmann grundsätzlich bekannte Lyocell-Verfahren, beschrieben unter anderem in der EP 0356419. In die Spinnmasse können während ihrer Herstellung, in jedem Fall aber vor der Extrusion, zusätzlich Stoffe inkorporiert werden, wie weiter oben bereits beschrieben. Ausgehend von der Celluloselösung erfolgt die Formgebung, bei der - insbesondere im Fällungsprozess - darauf zu achten ist, dass sich keine faserigen Strukturen ausbilden. Dies ist keine triviale Anforderung, da Cellulose aufgrund ihrer makromolekularen Struktur bestrebt ist, faserige Domänen auszubilden. Gelöst wird dieses Problem dadurch, dass die Celluloselösung zunächst ohne wesentliche Scherung in die gewünschte Form gebracht wird und anschließend auch die Regenerationsbedingungen entsprechend gewählt werden. Dabei ist unbedingt erforderlich, dass die Celluloselösung direkt, d. h. ohne Luftspalt, in ein Fällbad extrudiert wird und die Zerkleinerung des Lösungsstranges auf eine Weise stattfindet, die im Wesentlichen gleichgroße Partikel ergibt. Geeignete Aggregate für diesen Schritt sind etwa Unterwasser- oder Stranggranulatoren, womit sich neben sphärischen Partikeln auch Zylinder, Drehellipsoide und Ovoide erzeugen lassen. Die eben erwähnten Aggregate erfüllen auch die zusätzlichen Anforderungen an den Herstellungsprozess. Die erzeugten Partikel sollen nämlich eine möglichst einheitliche Größe aufweisen, wobei sich die Eigenschaften über die Verfahrensparameter steuern lassen. Gleichzeitig soll das Verfahren einen hohen Durchsatz aufweisen.

Granulate unterschiedlicher Größe können aus Lyocell-Spinnmasse beispielsweise mit einem Unterwassergranulator der Firma Econ vom Typ EUP50 hergestellt werden, wobei je nach Ausführung von Lochplatte und Düsenloch hohe Durchsätze von 2 bis 30 kg/h - gerechnet auf 100% Cellulose, NMMO-frei gewaschenes und getrocknetes Endprodukt - möglich sind. Die erzeugten Kügelchen können dabei über einen mechanischen Zentrifugaltrockner vom Prozesswasser getrennt werden. In anderen Ausführungen können solche Fest-Flüssig-Trennungen z.B. mittels Hydrozyklon, Schubzentrifugen oder auch durch Siebe bewerkstelligt werden. Granulatoren sind in verschiedenen Größen am Markt erhältlich und durch die Einfachheit des Verfahrens zur Granulierung von Spinnmasse ist die Hochskalierung auf großtechnischen Maßstab relativ einfach. So können z.B. mit einem einzigen Granulator des Typs EUP 3000 etwa 5000 Tonnen Kügelchen pro Jahr produziert werden. Weiters stehen von anderen Herstellern noch wesentlich größere Maschinen zur Verfügung.

In einer weiteren Ausführung können auch mit speziellen Lyocell-Düsen mit Düsenlochdurchmessern von 0,5 bis 5 mm Cellulosestränge gefertigt werden, die nach einer Waschstrecke einem Stranggranulator zugeführt werden. Kritisch in dieser Hinsicht sind das Auswaschen, die Zuführung und der Einzug der einzelnen Stränge in den Stranggranulator, da die Stränge sehr flexibel sind. Auf diese Weise können zylinderförmige Granulate erhalten werden.

Großen Einfluss auf die Eigenschaften der erhaltenen Partikel hat auch die Viskosität der Celluloselösung, da diese normalerweise auch die Viskositätsdifferenz zwischen Celluloselösung und Fällbad dominiert. Das Fällbad ist bevorzugt wässerig (mit einer Viskosität im Bereich von 1 Pa.s), allerdings kann durch Zugabe von Verdickern (Polymeren) auch die Viskosität des Fällbades deutlich erhöht werden. Ein geringerer Viskositätsunterschied führt zu einem dünneren Mantel. Erfindungsgemäß ist ein Viskositätsunterschied zwischen Celluloselösung und Fällbad von mind. 600 Pa.s, bevorzugt im Bereich 750 - 1200 Pa.s. (bezogen auf die Nullviskosität).

Die Dicke des Mantels wird entscheidend durch die NMMO-Konzentrationsdifferenz beim Eintritt der Celluloselösung in das Fällbad beeinflusst. Je größer diese ist, desto dicker wird der Mantel der erfindungsgemäß hergestellten Formkörper. Die Konzentrationsdifferenz wird maximal, wenn als Fällbad reines Wasser verwendet wird und das Fällbad an der Eintrittsstelle der Celluloselösung so gut durchmischt wird, dass sämtliches austretendes NMMO sofort wegtransportiert wird.

Die Dicke des Mantels wird auch durch die Temperaturdifferenz beim Eintritt der Celluloselösung in das Fällbad beeinflusst. Je größer diese ist, desto dicker wird der Mantel der erfindungsgemäß hergestellten Granulate.

Neben der Möglichkeit der Unterwassergranulierung in einem flüssigen Fällbad besteht auch die Möglichkeit der Koagulation in einem gasförmigen Medium.

Das bevorzugte Verfahrensprinzip für das Auswaschen der erfindungsgemäßen Formkörper im großtechnischen Maßstab ist die Gegenstromwäsche, um die notwendige Waschwassermenge und die Rückgewinnungskosten in Grenzen zu halten. Um die geforderte Reinheit der Formkörper zu erreichen, sind dafür 10 bis 12 Waschstufen notwendig. Bei geringen NMMO-Restgehalten ist außerdem die Erhöhung der Waschwassertemperatur vorteilhaft. Bevorzugt ist hier eine Waschwassertemperatur von 60°C bis 100°C. Um auch geringe Mengen an Abbauprodukten des Lösungsmittels effizient zu entfernen, ist zusätzlich ein alkalischer Schritt notwendig, wobei bevorzugt pH-Werte von 9-13 verwendet werden.

Als Verfahren für die erfindungsgemäße großtechnische Wäsche sind grundsätzlich insbesondere alle Arten von Fest-Flüssig-Extraktionen im kontinuierlichen oder Batch-Betrieb geeignet. Bevorzugt sind jedoch Verfahren nach dem Perkolationsprinzip, d.h. mit einer Kreuz-Gegenstromwäsche. Geeignete Vorrichtungen hierfür sind unter anderem Karussellextraktoren oder Extraktoren der Bauart De-Smet, Crown oder Bollmann. Auch Kaskaden sind dafür geeignet. Solche Extraktoren werden auch beispielsweise bei der Mazeration eingesetzt. Schubzentrifugen wären grundsätzlich ebenfalls geeignet, aber für das erfindungsgemäße Verfahren ist es wichtig, dass eine Scherung oder Druckbelastung der Granulate möglichst vermieden wird, so dass Zentrifugen eher nicht in Frage kommen. Weiters geeignet sind Säulen nach dem lonentauscherprinzip, wobei das Lösemittel für Cellulose vorzugsweise über die Säule von oben nach unten verdrängt wird. Auch diese können wieder zu Kaskaden angeordnet werden.

Im Hinblick auf die vielfältigen Verwendungsmöglichkeiten der erfindungsgemäß hergestellten Produkte ist es wichtig, das NMMO möglichst restlos zu entfernen, da NMMO eine oxidierende Wirkung auf einige Wirksubstanzen haben kann, die später in die Produkte eingebracht werden.

Nach der Granulatwäsche sollte die überschüssige anhaftende Feuchte von den Partikeln entfernt werden, um die Trocknungskosten zu minimieren und das Granulat rieselfähig zu machen. Geeignete Aggregate dafür sind Zentrifugen und Dekanter sowie Bandfilter die kontinuierlich oder diskontinuierlich betrieben werden können.

Als zusätzlicher Prozessschritt kann nach der Wäsche des Granulats auch noch eine Dampfsterilisierung folgen. Durch die Dampfsterilisierung geht das Wasserrückhaltevermögen der niemals getrockneten Celluloseperlen zurück und die Vorentwässerung vor der Trocknung kann effizienter erfolgen.

Im Hinblick auf den Einsatz in der Kosmetik, wo viele Produkte wässerige Formulierungen oder Emulsionen sind, sind niemals getrocknete Partikel mit einer definierten Feuchte eine bevorzugte Ausführungsform dieser Erfindung. Dadurch wird die nach der Regeneration und dem Auswaschen des Lösungsmittels vorhandene, sehr offene Porenstruktur erhalten und die Cellulosepartikel sind sehr aufnahmefähig und zugänglich.

Weiters bildet sich durch die Koagulation der Spinnmasse unter Zuhilfenahme der oben genannten Aggregate eine Kern/Mantel-Struktur aus. Diese Kern/Mantel-Struktur zeigt sich anhand einer kompakten transparenten Außenhaut (Mantel) sowie einem schwammartigen weißen Inneren (Kern) der Kügelchen.

Diese Struktur ist für die Controlled-Release-Eigenschaften der erfindungsgemäßen Formkörper bei der Abgabe von kosmetischen oder pharmazeutischen Wirkstoffen verantwortlich, da die Wirkstoffe, welche im schwammartigen Inneren leicht verfügbar vorliegen, bei der Abgabe die sehr kompakte Außenhaut überwinden müssen. Die Abgabe der Wirkstoffe wird dadurch verzögert. Die Dicke und Struktur dieser Außenhaut der Kügelchen kann durch Veränderung der Fällmediumparameter verändert werden.

Zwischen Schritt d. und Schritt e. kann eine Enzymbehandlung stattfinden, um dem erfindungsgemäßen Formkörper entsprechende funktionale Eigenschaften zu verleihen, wie im Folgenden ausführlich beschrieben wird. Hierfür wird bevorzugt eines oder mehrere Enzyme, ausgewählt aus der Gruppe enthaltend Exo- und Endo-1,4-b-glucanasen, Glucosidasen und Xylanasen, eingesetzt. Überraschenderweise wird bei einer Enzymbehandlung der erfindungsgemäßen Partikel nicht nur die Oberfläche angegriffen (diese wird geglättet), sondern auch die poröse Struktur im Inneren. Daraus lässt sich schließen, dass Enzyme in das Innere des Partikels einwandern können. Es lässt sich mittels Enzymbehandlung somit erfindungsgemäß die Festigkeit des Partikels stufenlos einstellen. Weiters zeigt dies, dass Enzyme oder Proteine ins Innere der Kügelchen eindringen können und eine Beladung der auf diese Weise leichter zugänglichen Kügelchen mit diesen Substanzen möglich ist. Die Enzymbehandlung der Kügelchen zeigt auch, dass sowohl Enzyme als auch Proteine von den Kügelchen aufgenommen werden können. Bei entsprechender Trocknung können diese auch verkapselt bzw. immobilisiert werden.

Die Bildung dieser Kern/Mantel-Struktur ermöglicht es, auch Kügelchen mit sogenannten Stimuli-Response-Eigenschaften herzustellen. So lässt sich einerseits die Elastizität der Kügelchen durch die variierbare Ausformung der Außenhaut drastisch verändern, andererseits lässt sich jedoch sowohl das Innere der Kügelchen als auch dessen Außenhaut durch chemische oder enzymatische Modifikation oder die Variation der Fällmediumparameter so verändern, dass die Kügelchen bereits bei geringem Druck zwischen den Fingern aufplatzen und damit gezielt ihren Inhalt freisetzen (beispielsweise ein Hydrogel ausbilden). Das durch das Aufplatzen der Kügelchen entstehende Hydrogel weist sehr interessante kosmetische Eigenschaften auf. So ist keinerlei Klebrigkeit, Schmierigkeit oder Öligkeit dieses Hydrogels feststellbar. Auch dessen Partikelgröße und Körnigkeit kann durch die Dauer der chemischen Modifikation, enzymatischen Behandlung oder auch Änderung der Fällbadparameter individuell eingestellt werden. Dadurch können die Kügelchen auch als Texturbildner in der Kosmetik verwendet werden.

Dieser Stimuli-Response-Effekt ist für den Kosmetik- bzw. Pharmasektor von besonderer Bedeutung, da z.B. kosmetische oder pharmazeutische Wirkstoffe, aber auch Farbpigmente bzw. Farben der dekorativen Kosmetik, welche in die Kügelchen eingebracht wurden, dadurch gezielt freigesetzt werden können. Weiters können die Kügelchen als Peeling oder Exfoliants mit Stimuli-Response-Effekt eingesetzt werden. Dabei können diese Kügelchen im Inneren abrasive Pigmente oder auch Enzyme enthalten, welche durch das Aufplatzen beim Verreiben der Kügelchen freigesetzt werden können und dadurch den gewünschten Peeling-Effekt erzielen. Der Stimuli-Response-Effekt der Kügelchen kann durch die Dauer der chemischen Modifikation, der enzymatischen Behandlung oder auch durch Änderung der Fällmediumparameter individuell eingestellt werden.

Sollten für bestimmte Anwendungen Partikel mit nur geringem Feuchtegehalt von Vorteil sein, können die erfindungsgemäßen Partikel auch mittels verschiedener Trocknungsverfahren getrocknet werden. In einer bevorzugten Ausführungsform des erfindungsgemäßen Verfahrens erfolgt der Trocknungsprozess durch NormaldruckTrocknung, Strömungstrocknung, Wirbelschichttrocknung, Gefriertrocknung oder superkritischer CO2-Trocknung. Die Trocknung ist insofern eine Herausforderung, da die Produktfeuchte sehr hoch ist. Bei den Celluloseperlen müssen Gutsfeuchten von 70-75 Gew.-% bewältigt werden, wobei bis zur Gleichgewichtsfeuchte von 10 - 13 Gew.-% bzw. für manche Anwendungen auf < 5 Gew-% getrocknet werden muss. Zur Schonung des Produktes sollte die Trocknung erfindungsgemäß möglichst kontaktlos erfolgen.

Überraschenderweise hat sich die Trocknung im Wirbelschichtverfahren als besonders schonend und effizient erwiesen. Durch die permanente Umwälzung und Auflockerung des Gutes ist das Austreiben der Feuchtigkeit stark begünstigt. Außerdem ist die Trocknung sehr produktschonend hinsichtlich Abrieb. Zusätzlich können hohe Durchsätze und kurze Trocknungszeiten erreicht werden. Diese wiederum führen dazu, dass die Vergilbung, wie sie durch Einwirkung von erhöhten Temperaturen normalerweise auftritt, äußerst gering ist. Der Prozess kann zudem kontinuierlich betrieben werden. Hohe Schüttungen des Gutes, wie sie teilweise in anderen Trocknungsverfahren auftreten, würdendazu führen, dass die Feuchtigkeit sehr lange zwischen den Körnern verweilt, wodurch die Trocknungszeit immens ansteigt und die lange Temperatureinwirkung zusätzlich die Gefahr der Vergilbung mit sich bringt. Als Beispiele wären hier etwa Konustrockner oder Kristallisatoren zu nennen. Durch das Umrühren entsteht in diesen Aggregaten außerdem eine hohe Scherung am Produkt, wodurch die äußere Haut abgerieben wird und sehr viel Staub entsteht. Weiters wird durch diesen Abrieb auch die Dicke der Mantelschicht verändert bzw. diese zerstört, wodurch die Controlled-release- Eigenschaften des Produkts vermindert bzw. aufgehoben würden. Diese Aggregate sind für das erfindungsgemäße Verfahren daher nicht geeignet.

Als erfindungsgemäße Alternative zur Wirbelschichttrocknung können noch Vibrationstrockner gesehen werden, die ähnliche Vorteile in der Trocknung bringen. Diese können für bestimmte inkorporierte Produkte von Vorteil sein, da hier das Trocknen unter Vakuum bei niedrigen Temperaturen erfolgt.

Ein weiterer Vorteil des Wirbelschichtverfahrens ist, dass dabei in einem Prozessschritt sowohl getrocknet als auch mit einem zusätzlichen Stoff beschichtet ("gecoatet") werden kann. Die Möglichkeiten im Coatingprozess sind vielfältig. Beispielsweise können die Granulate eingefärbt werden oder es können funktionelle Stoffe aufgebracht werden wie beispielsweise Biopolymere (z.B. Chitosan etc.), synthetische Polymere, aktive kosmetische oder pharmazeutische Wirkstoffe, Enzyme, Proteine und Trennmittel sowie Mahlhilfsmittel. Auch eine chemische Modifikation der Oberfläche der Kügelchen in der Wirbelschicht ist möglich. Durch das Coaten in der Wirbelschicht ist die Verteilung des Coatings über die Oberfläche und über die einzelnen Partikel sehr homogen. Überraschenderweise hat sich gezeigt, dass die beschichteten, mittels Wirbelschicht getrockneten erfindungsgemäßen Formkörper weiterhin ein sehr gutes Quellverhalten aufweisen.

Durch Auswahl von entsprechenden Trocknungsverfahren lässt sich auch die Porenstruktur und die Dichte der erfindungsgemäßen Formkörper erheblich beeinflussen. Bei einer Normaldrucktrocknung bei 60°C in einem Umlufttrockenschrank kollabiert die schwammartige Struktur der niemals getrockneten Kügelchen vollständig und es entsteht ein nahezu transparentes kompaktes, sehr viel kleineres Kügelchen, das aber seine ellipsoide Form beibehält. Dieser Effekt des Kollabierens der Struktur im Inneren der Kügelchen ist jedoch zum Teil reversibel, da die Kügelchen in Wasser wieder quellen. Bei Normaldruck zeigte die Wirbelschichttrocknung, dass die Schwammstruktur der niemals getrockneten Kügelchen bei dieser am besten durch anschließendes Quellen in Wasser wieder herstellbar war - wie bereits oben ausgeführt.

Die Schwammstruktur konnte jedoch um einiges besser erhalten werden, wenn die Kügelchen durch Schockgefrieren der niemals getrockneten Kügelchen in flüssigem Stickstoff und anschließendes Gefriertrocknen hergestellt wurden. Hierbei zeigte sich, dass diese getrockneten Kügelchen nicht mehr transparent, sondern weiß waren. Dies ist ein Indiz für den Erhalt der Porenstruktur. Die Oberfläche und Form der Kügelchen veränderte sich jedoch sehr stark während der Trocknung. Das erhaltene Produkt bewahrte zwar im Wesentlichen seine ellipsoide Form, jedoch hatten sich auf der Oberfläche Dellen und Krater ähnlich einer Mondlandschaft gebildet. Das Kügelchen zeigte damit ein rosinenähnliches Aussehen. Weiters war die Dichte dieser Kügelchen geringer als Wasser.

Als weitere Trocknungsart wurde die superkritische CO₂-Trocknung bei vorangegangenem Lösungsmitteltausch für die Trocknung der niemals getrockneten Kügelchen ausgewählt. Im Besonderen wurde hier das Wasser in den Kügelchen gegen Aceton ausgetauscht und danach die superkritische CO₂-Trocknung gestartet. Bei dieser Trocknungsmethode konnte die ursprüngliche Form und Porosität der niemals getrockneten Kügelchen am besten erhalten werden. Diese hatten eine ellipsoide Form mit glatter Oberfläche. Die feine Porenstruktur zeigte sich durch die weiße Farbe der Kügelchen. Auch diese Kügelchen hatten eine geringere Dichte als Wasser.

Ein weiterer Gegenstand der vorliegenden Erfindung ist auch die Verwendung der erfindungsgemäßen Formkörper; aufgrund ihrer einzigartigen Struktur können sie mit verschiedensten Wirkstoffen beladen werden.

Erfindungsgemäß können die Formkörper zur Herstellung eines wirkstoffbeladenen Trägermaterials verwendet werden, wobei der Formkörper mit einer Lösung des Wirkstoffes getränkt und anschließend gewaschen und getrocknet wird.

Weiterhin können sie zur Herstellung eines wirkstoffbeladenen Trägermaterials mit Controlled-Release-Eigenschaften für kosmetische und pharmazeutische Anwendungen verwendet werden. Hierzu ist unter Umständen eine vorherige Mahlung der erfindungsgemäßen Formkörper notwendig, wobei die Controlled-Release-Eigenschaften zu einem Teil erhalten bleiben; besonders bevorzugt sind dabei folgende Anwendungen: Naturstoffemulsionen, Gelemulsionen, Herrenpflegeprodukte, Gesichtspflegeprodukte, Sonnenschutzprodukte, kosmetische Seren, Desodorierende Applikationen, Make-up-Grundlagen und Farbkosmetik. Die Wirkstoffe können beispielsweise Enzyme und Peptide für kosmetische und technische Anwendungen sein, wie z.B. das Coenzym Q10.

Erfindungsgemäß können die Formkörper auch als abrasives Material in kosmetischen Produkten wie zum Beispiel Peelings oder Exfoliator-Mischungen verwendet werden, wobei die durchschnittliche Größe der Formkörper 150 - 800 µm, bevorzugt 200 - 800 µm, besonders bevorzugt 300 - 550 µm beträgt.

Ebenso können die Formkörper erfindungsgemäß als optische Effektkügelchen in kosmetischen Produkten, bevorzugt Shampoos und Cremes verwendet werden.

Die Formkörper können sowohl im nie getrockneten als auch im getrockneten Zustand erfindungsgemäß auch als Ausgangsmaterial zur Herstellung von sphärischen cellulosischen Pulvern, die Sensoric-Booster-Eigenschaften in Öl/Wasser-Emulsionen aufweisen, verwendet werden. Bevorzugt werden die Formkörper hierbei mittels verschiedener Mahlmethoden auf Teilchengrößen von bevorzugt d₅₀ = 5 µm gemahlen. Sie werden dann für kosmetische und Körperpflegeprodukte eingesetzt. Ihre Vorteile bestehen vor allem darin, daß die Endprodukte weniger Klebrigkeit und Schmierigkeit sowie ein verbesserte Absorption von Lotionen in die Haut aufweisen. Besonders bevorzugt sind dabei Naturstoffemulsionen, Gelemulsionen, Herrenpflegeprodukte, Gesichtspflegeprodukte, Sonnenschutzprodukte, kosmetische Seren, Desodorierende Applikationen, Make-up-Grundlagen und Farbkosmetik.

Im technischen Bereich können die oben beschriebenen Formkörper erfindungsgemäß als Säulenmaterial in der Chromatographie, insbesondere in der Normalphasen-, Umkehrphasen-, lonenaustausch-, Affinitäts- und Größenausschlußchromatographie verwendet werden Hierfür können die Formkörper auch chemisch modifiziert werden, beispielsweise mittels Acetylierung, Methoxylierung, oder ähnlicher Verfahren.

Weiterhin können die oben beschriebenen Formkörper erfindungsgemäß zur Immobilisierung von Enzymen, oder Peptiden verwendet werden, um deren enzymatische Aktivität oder Stabilität zu erhöhen. Besonders bevorzugt hierfür ist der Einsatz im Kosmetik- und technischen Bereich. Ebenso können die oben beschriebenen Formkörper erfindungsgemäß zur Immobilisierung von Zellen menschlichen, tierischen oder pflanzlichen Ursprungs (Bakterien, Pilze, Gewebe, Algen, etc.) verwendet werden.

Die oben beschriebenen Formkörper können auf verschiedene Weise handhabbar gemacht werden. Eine erfindungsgemäß bevorzugte Variante ist das Einbringen in Vliesstoffe (auch "Nonwovens" genannt). Die Vliesstoffe selbst können dabei nach den im Stand der Technik bekannten Verfahren wie Kardieren, Spunbonding, Meltblowing oder Air-laid-Verfahren hergestellt sein. Das Einbringen kann in den verschiedenen Stufen der Herstellung des Vliesstoffes und/oder auf den fertigen Vliesstoff erfolgen:
a. Vor der Vliesbildung: In Air-laid und Wet-laid-Verfahren können die erfindungsgemäßen Formkörper vor der Vliesbildungsstufe mit den übrigen Ausgangsmaterialien gemischt werden. In Wet-laid-Verfahren können sie beispielsweise in Flüssigkeiten oder Schaum dispergiert werden.
b. Während der Vliesbildung: In Extrusionsverfahren wie Meltblowing- oder Spunbond-Verfahren können die erfindungsgemäßen Formkörper während oder direkt nach der Filamentformung zugegeben werden, wenn die Filamentstränge auf dem Siebband oder der Siebtrommel abgelegt werden. Das Einbringen der Formkörper kann dabei z.B. durch Einsprühen in den Filamentvorhang oder Aufsprühen vor bzw. nach dem Ablegen des Filamentvorhangs auf Siebband bzw. Siebtrommel erfolgen.
c. Nach der Vliesbildung, aber vor der Verfestigungsstufe.
d. Nach der Verfestigungsstufe (online), wobei die Verfestigung mittels bekannter Verfahren aus dem Stand der Technik erfolgen kann, beispielsweise mittels Thermobonding, chemischer Vernetzung, Air-Through-Bonding, Ultrasonic bonding, Needlepunching, Spunlacing und auch Kombinationen von zwei oder mehreren dieser Verfahren.
e. Auf vorverfestigten Vliesstoffen, beispielsweise vor dem Aufbringen einer zweiten Vliesstoffschicht. Dabei kann der vorverfestigte Vliesstoff beispielsweise auch in einem diskontinuierlichen Verfahren von einer Rolle aus zugeführt werden.
f. In Verfahren, die eine Kombination der oben beschriebenen Varianten a. bis e. darstellen.

Die erfindungsgemäßen Formkörper werden in allen diesen Verfahren bevorzugt mittels eines Bindemittels, beispielsweise einem Klebstoff fixiert.

Mit diesen Verfahren lassen sich aus den erfindungsgemäßen Formkörpern (A) und Vliesstoffen (B) die unterschiedlichsten Schichtstrukturen herstellen: Einseitig (A-B) oder auch beidseitig (B-A-B) mit Formkörpern beschichtete Vliesstoffe, Sandwichstrukturen aus zwei Vliesstoffen mit innenliegender Formkörper-Schicht (AB-A) oder auch mehrfach geschichtete Systeme, die abwechselnd Vliesstoff- und Formkörper-Schichten aufweisen (A-B-A-B, A-B-A-B-A, ...).

Eine ebenfalls mögliche Variante ist das Aufbringen der erfindungsgemäßen Formkörper auf Kunststofffolien. Das Fixieren der Formkörper erfolgt dabei bevorzugt ebenfalls mittels Klebstoff.

### Beispiele

Die folgenden Beispiele dienen der Illustration und dem besseren Verständnis der erfindungsgemäßen Partikel sowie deren Herstellungsweise. Die Erfindung selbst ist jedoch nicht nur auf diese exemplarischen Ausführungsformen beschränkt.

### Beispiel 1 - Herstellung von Celluloseperlen

Als Ausgangsmaterial zur Herstellung der Celluloseperlen wurde eine Standard-Lyocellspinnmasse mit folgender Zusammensetzung verwendet: 13 Gew.-% Zellstoff (100 % Saiccor), 75,3 Gew.-% NMMO, 11,7 Gew.-% Wasser sowie Spuren an Stabilisator. Die Spinnmasse wurde auf 120-125°C gehalten und mittels Unterwassergranulierer ECON EUP 50 verarbeitet. Es wurden dabei durchgängige Lochplatten mit je 12 Löchern und 4 Messer verwendet. Anfahrventil und Lochplatte wurden auf konstant 120°C temperiert, der Wassertank auf 20°C. Tabelle 1 fasst jene Parameter zusammen, die während der Versuche V1 bis V6 variiert wurden.

**Tabelle 1**

| | V1 | V2 | V3 | V4 | V5 | V6 |
|---|---|---|---|---|---|---|
| Lochdurchmesser [mm] | 1,0 | 1,5 | 1,5 | 1,5 | 1,5 | 1,5 |
| Messerdrehzahl [rpm] | 4500 | 3000 | 3000 | 3000 | 3000 | 3000 |
| Temperatur Fällbad [°C] | 30 | 50 | 50 | 50 | 50 | 10 |
| Konz. NMMO Fällbad [Gew.-%] | 20 | 34,5 | 38,9 | 42 | 44,6 | 0 |
| Hautdicke [µm] - Mittelwert | 114,7 | 89,0 | 85,0 | 75,2 | 53,6 | 182,2 |
| Hautdicke [µm] - Standardabweichung | 20,1 | 14,7 | 4,2 | 7,7 | 8,0 | 35,3 |

Die mittels Unterwassergranulierer hergestellten Celluloseperlen wurden in reinem vollentsalzten Wasser (VE-Wasser) ausgefällt und anschließend mit 80°C heißem VE-Wasser einer Wäsche in einer Säule unterzogen. Die Feinwäsche erfolgte mit Natronlauge (pH 11). Nach der Natronlauge-Wäsche wurden die Kügelchen mit VE-Wasser mit einer Temperatur von 80°C neutral gewaschen und bis zu einer Restfeuchte von etwa 70 Gew.-% abgeschleudert.

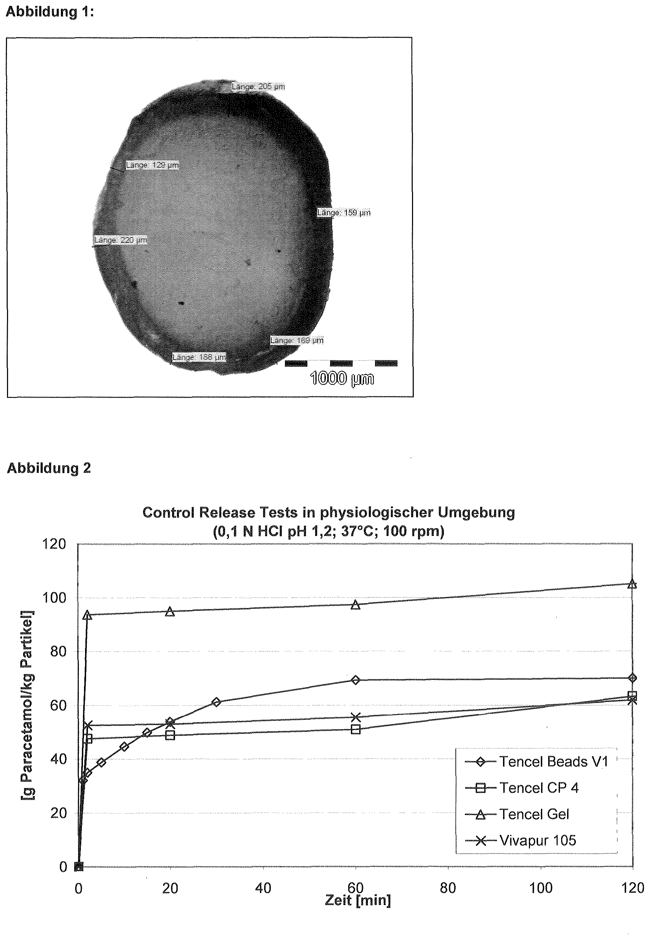

Die Struktur der niemals getrockneten Partikel aus den Versuchen V1 bis V6 wurde unter einem optischen Mikroskop (Typ Zeiss Discovery.V12, Olympus DP71) untersucht. Dabei zeigte sich, dass alle Partikel eine ausgeprägte Kern-Hülle-Struktur aufweisen. Abbildung 1 zeigt als Beispiel ein Partikel aus V6.

Mittels der Software AnalySIS 5.0 von Olympus wurde die Dicke der Hülle vermessen, wobei fünf Messungen durchgeführt und gemittelt wurden. Die Ergebnisse sind ebenfalls in Tabelle 1 zusammengefasst. Es wurde eine deutliche Abhängigkeit der Manteldicke von den Regenerationsbedingungen gefunden. Mit steigender NMMO-Konzentration im Fällbad bzw. mit Erhöhung der Temperatur nimmt die Dicke der äußeren Schicht ab.

Weiters ist zu erkennen, dass die Außenhaut transparent ist, während das Innere der Kügelchen weiß ist. Dies zeigt eindeutig die unterschiedliche Strukturierung des Materials. Die transparente Schicht ist sehr dicht, wohingegen das weiße Innere der Celluloseperlen eine schwammähnliche Struktur aufweist. Eine ausführliche Charakterisierung der Kern-Hülle-Struktur ist bei Beispiel 5 zu finden.

### Beispiel 2 - Beladung mit Aktivsubstanz

20 g niemals getrocknete Celluloseperlen V1 aus Beispiel 1 (68,3 Gew.-% Restfeuchte) wurden 40 ml einer auf 30°C temperierten 5 Gew.-% Paracetamol (Merck) in Ethanol p.A. Lösung hinzugefügt. Diese Suspension wurde bei 100 rpm für 120 min gerührt. Die beladenen Celluloseperlen wurden abgenutscht und mit 10 ml vollentsalztem (VE-)Wasser nachgewaschen sowie anschließend im Vakuumtrockenschrank bei 40°C, 150 mbar für ca. 8 h getrocknet. Die Auswaage der getrockneten Celluloseperlen betrug 5,6915 g. Die Beladung der Celluloseperlen mit Paracetamol betrug 8,48 Gew.-% (Gewichtszunahme).

Als Vergleichssubstanzen wurden folgende Materialien verwendet: Tencel® CP 4 (sphärisches Cellulosepulver, Hersteller Lenzing AG), Tencel® Gel (Cellulosesuspension, Lenzing AG) und Vivapur 105 (MCC, Hersteller J. R & Söhne). Hieraus wurden ebenfalls Suspensionen mit Paracetamol in Ethanol analog den erfindungsgemäßen Celluloseperlen hergestellt, wobei das Verhältnis Cellulose zu Paracetamol stets konstant gehalten wurde. Die Vergleichsmaterialien wurden allerdings nicht im Vakuumtrockenschrank getrocknet, da dies aufgrund der feinen Partikel zu Verklumpungen geführt hätte, sondern mittels Sprühtrockner Büchi B290. In Tabelle 2 sind die Eigenschaften der beladenen Partikel zusammengefasst.

**Tabelle 2**

| Material | Perlen V1 | Tencel® CP4 | Tencel® Gel | Vivapur 105 |
|---|---|---|---|---|
| d₅₀[µm] | 1260 | 7,5 | 6,3 | 10,5 |
| Beladung [Gew.-%] | 8,48 | 5,17 | 10,05 | 5,23 |

### Beispiel 3 - Release-Versuche

Die Wiederfreisetzungsrate von Paracetamol aus den in Beispiel 2 hergestellten beladenen Partikeln wurde gemäß der im Folgenden beschriebenen Methode bestimmt. Dazu wurden 100 mg der Partikel in 500 ml wässrige Salzsäure (0,1 M; pH 1,2) eingebracht und bei 37°C mit 100 rpm gerührt (Rührgerät: Erweka Dissolution Tester DT 820). In regelmäßigen Abständen wurden Proben entnommen und die Extinktion bei 243 nm gegenüber einer Referenz (reine wässrige Salzsäure) gemessen (Perkin Elmer Lambda 950 UV/VIS Spektrometer). In Abbildung 2 sind die Freisetzungskurven der verschiedenen Partikel dargestellt.

Es zeigt sich eindeutig, dass die erfindungsgemäßen Celluloseperlen eine signifikant retardierende Wirkung auf den Wirkstoff Paracetamol zeigen. Diese retardierende Wirkung ist im Vergleich zu pulvrigen Partikeln, die durch Sprühtrocknung beladen werden, sehr viel stärker ausgeprägt. Bei den Partikeln, die aus dem Cellulose-Gel hergestellt wurden, kann aufgrund der nochmals offeneren Struktur die höchste Beladung erreicht werden. Allerdings erfolgt auch hier die Freisetzung schlagartig; nur die erfindungsgemäßen Celluloseperlen zeigen aufgrund ihrer inneren Struktur slow-release-Eigenschaften.

### Beispiel 4 - Trocknung (inkl. Coating)

Die niemals getrockneten Celluloseperlen V1 aus Beispiel 1 (Restfeuchte 63,83%) wurden unterschiedlichen Trocknungsverfahren ausgesetzt, um deren Einfluss auf die Struktur der getrockneten Partikel zu untersuchen:
- Trockenschrank bei 60°C
- Wirbelschichttrocknung (in einem Wirbelschichttrockner DMR WFP-8) bei 100°C
- Trocknung mit superkritischem CO₂ (5 I-Laboranlage Natex) - nach Lösungsmitteltausch zu Aceton ("sc-CO₂-Trocknung")
- Gefriertrocknung (Labconco Freezone 2.5 Liter, Vakuum 0 mbar) nach Schockgefrieren in flüssigem N₂

Bei den Wirbelschichttrocknungen wurden zusätzlich noch Coatingversuche durchgeführt. Dazu wurden entsprechende wässerige Farbstofflösungen (Waco blau, Waco pink, SepiCoat 3213 Yellow + Sepifilm Gloss, Sepicoat 3404 Green + Sepifilm Gloss, Sepicoat 5901 Brown + Sepifilm Gloss) in den Wirbelschichttrockner eingedüst. Alle erhaltenen Partikel zeigten eine homogene Färbung an der Oberfläche.

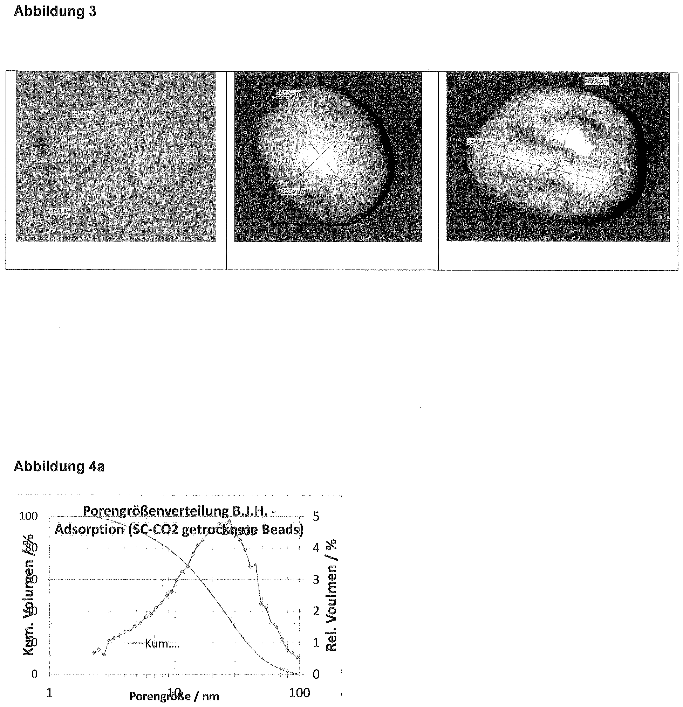

Bei allen Trocknungsverfahren konnte eine Schrumpfung der Partikel festgestellt werden. So hatten etwa die Celluloseperlen nach Trocknung im Trockenschrank eine Schüttdichte von 0,74 g/ml, während die niemals getrockneten Celluloseperlen noch eine Schüttdichte von 0,72 g/ml hatten. Die Zunahme der Schüttdichte weist direkt auf eine Abnahme der Partikelgröße hin. Abbildung 3 zeigt typische Strukturen, die aus den verschiedenen Trocknungsverfahren erhalten wurden:
- Links: Trockenschrank - transparente bis leicht opake Celluloseperle mit rauer Oberfläche
- Mitte: sc-CO₂-Trocknung weiße Celluloseperle mit glatter Oberfläche
- Rechts: Gefriertrocknung - weiße Celluloseperle mit glatter, aber verformter Oberfläche

Das Trocknungsverfahren hat aber nicht nur Einfluss auf das äußere Erscheinungsbild der Partikel, sondern auch auf deren innere Struktur. Dazu wurde die BET-Oberfläche der Partikel mittels N₂-Adsorption (Meßgerät BELsorp mini II) bestimmt. Für die im Trockenschrank getrockneten Celluloseperlen konnte mit dieser Messung keine BET-Oberfläche bestimmt werden, was bedeutet, dass die innere Porenstruktur vollständig kollabiert ist. Für die sc-CO₂-getrocknet Probe wurde eine BET-Oberfläche a_{BET} = 174 m²/g erhalten, was auf einen weitgehenden Erhalt der im niemals getrocknetem Zustand vorhandenen Porenstruktur hinweist. Mit a_{BET} = 45 m²/g liegt das gefriergetrocknete Muster erwartungsgemäß zwischen den beiden anderen Verfahren. In der Abbildung 3a. wird weiters die Porengrößenverteilung der sc-CO₂-getrockneten Celluloseperlen anhand eines B.J.H. Plots (berechnet aus der N₂-Ad-/Desorption) gezeigt, die hauptsächlich im Bereich von 25 nm liegt.

Weiters konnte durch Heliumpyknometrie (Pycnomatic ATC der Firma ThermoFisher/Porotec) bewiesen werden, dass die sc-CO₂-getrockneten Celluloseperlen eine offenporige Struktur aufweisen wohingegen die gefriergetrockneten Celluloseperlen eine geschlossenporige Struktur zeigen.

### Beispiel 5 - Charakterisierung der Kern-Hülle Struktur

An den niemals getrockneten Proben V4 und V6 aus Beispiel 1, die eine deutlich unterschiedliche Dicke der Hülle aufweisen (75 µm bzw. 182 µm), wurden C¹³ CP-MAS-NMR Messungen durchgeführt (Spektrometer Bruker Avance DPX 300 NMR, 7.05T Magnetfeldstärke Ultrashield (SB), 2 rf-Kanäle, 100/300 W ¹H/BB Verstärker, 4 mm ¹H/BB Festkörper CP-MAS Probenkopf). Aus diesen Messungen wurde nach der in "G. Zuckerstätter et. al., Novel insight into cellulose supramolecular structure through 13C CP-MAS NMR spectroscopy and paramagnetic relaxation enhancement, Carbohydrate Polymers 93 (2013), p. 122 - 128" beschriebenen Methode die innerkristallinen (IC), oberflächenkristallinen (SC) und ungeordneten Anteile (DIS) bestimmt.

An Hand der in Abbildung 4 dargestellten Ergebnisse lässt sich eindeutig erkennen, dass sich durch ein Absenken der Fällbadtemperatur von 50°C (V4) auf 10°C (V6) und ein Absenken der NMMO-Konzentration im Fällbad von 44,6 Gew.-% NMMO (bei V4) auf 0 Gew.-% NMMO (bei V6) die ungeordneten Bereiche von 37,4 % auf 29,3 % erhöhen. Dadurch muss der innerkristalline Teil entsprechend abnehmen, da der oberflächenkristalline Anteil konstant bleibt. Daher kann man sagen, dass die Dicke der Aushaut mit den ungeordneten Anteilen korreliert und man kann die Außenhaut eindeutig als amorphen Teil des Kügelchens identifizieren. Durch Computertomographie-Messungen lässt sich eindeutig feststellen, dass diese amorphe Außenhaut aus dichterem Material als der Kern besteht.

Diese Röntgen-Computertomographie-Messungen wurden mit einem Gerät GE Phoenix / x-ray Nanotom und einer Voxelgröße von 4,5 pm durchgeführt, die Messdauer betrug 121 min und es wurden insgesamt 1700 Projektionen aufgenommen. Vermessen wurden ganze Celluloseperlen (nicht zerkleinert): Die gefriergetrockneten und mittels sc-CO₂-getrockneten Celluloseperlen aus Beispiel 4 sowie als Referenz kommerziell erhältliche kompakte Cellulose-Kügelchen (Sprayspheres-SE White, Umang). Eine Messung der feuchten Celluloseperlen war nicht sinnvoll möglich. Mittels der Software VG Studio MAX 2.2 wurden (virtuelle) Schnitte durch die Partikel gemacht und nach den Graustufen (entspricht der Streudichte) ausgewertet. Dabei wurde für Luft (Partikelzwischenräume) eine relative Dichte von 0% und für kompakte Cellulose (Sprayspheres) eine relative Dichte von 100% definiert. Tabelle 3 zeigt die Werte, die sich für die getrockneten Tencel®-Perlen ergeben.

**Tabelle 3**

| | relative Dichte Hülle | relative Dichte Kern |
|---|---|---|
| Tencel(R)-Perlen gefriergetrocknet | 71,8% | 54,4% |
| Tencel(R)-Perlen sc-getrocknet | 72,8% | 26,0% - 42,2% |
| Sprayspheres SE | 100% (homogen, Referenz) | |

### Beispiel 6 - Quellverhalten getrocknete Celluloseperlen

Die mittels Wirbelschicht getrockneten (ungecoateten) Celluloseperlen aus Beispiel 4 wurden auf ihr Quellverhalten in Wasser hin untersucht. Als Referenzmaterial wurden wiederum kommerzielle, mittels Sprühgranulierung hergestellte Cellulose-Kügelchen (Sprayspheres-SE White, Umang) verwendet. Je 10 g Partikel wurden in 100 ml VE-Wasser gegeben und ohne weitere Bewegung (Rühren o. ä.) quellen gelassen, bis sich ein Gleichgewicht einstellte. In Tabelle 4 sind die Ergebnisse dieser Versuche zusammengefasst.

**Tabelle 4**

| | Startfeuchte [Gew.-%] | Größe d₅₀ [µm] | Quelldauer [min] | Schüttquellung [vol%] | Feuchteaufnahme [Gew.-%] |
|---|---|---|---|---|---|
| Tencel Beads | 9,9 | 1207 | 180 | 170 | 99 |
| Sprayspheres | 8,7 | 734 | 40 | 90 | 63 |

Es zeigt sich, dass die erfindungsgemäßen Tencel-Beads ein deutlich besseres Quellverhalten aufweisen als vergleichbare kommerzielle Cellulose-Partikel. Das kann wiederum auf die bereits in den vorherigen Beispielen beschriebene innere Struktur zurückgeführt werden.

### Beispiel 7 - Variation der Festigkeit der Partikel durch enzymatische und chemische Behandlung

Die niemals getrockneten Celluloseperlen V1 aus Beispiel 1 wurden sowohl einer enzymatischen als auch einer oxidativen Behandlung unterzogen, wobei folgende Parameter zum Einsatz kamen:
In einer Pufferlösung (pH 4) wurde eine Lösung der Enzyme Celluclast 1.5L und Econase HC 400 (Novozyme) im Verhältnis 2:1 hergestellt. Eine weitere Möglichkeit der Enzymbehandlung ergibt sich durch den Einsatz von Novozyme FiberCare R oder FiberCare D in einer Pufferlösung mit pH 6,5. Die Celluloseperlen wurden jeweils in einer Pufferlösung (40°C) vorgelegt und anschließend die jeweilige Enzymlösung in einer Dosierung entsprechend 1 mL pro Gramm trockene Celluloseperlen zugegeben. Die Reaktionszeit betrug 10 min und 60 min, danach wurde die Reaktion durch Enzymdeaktivierung abgestoppt und die Celluloseperlen mehrfach mit VE-Wasser gespült.

Die Oxidation erfolgte über die bekannte TEMPO-Reaktion, wobei die Reaktionszeit hier 4 h bei 80°C betrug. Nach Stoppen der Reaktion durch EtOH-Zugabe und gründlichem Waschen der Celluloseperlen mit VE-Wasser erfolgte für 48 h noch eine Nachoxidation in 0,1 M Na-Acetat Puffer (pH=4,5). Nach der Reaktion wurden die Partikel abgenutscht, gründlich gewaschen und im Kühlschrank gelagert.

Die Festigkeit der Celluloseperlen wurde mittels verschiedener Stempel unterschiedlichen Gewichts, die auf eine definierte Menge Celluloseperlen aufgesetzt wurden, untersucht. Weiters wurden erneut Mikroskopieaufnahmen der Partikel angefertigt, um Änderungen in der Morphologie zu analysieren.

Enzymatisch behandelte, niemals getrocknete Celluloseperlen (10 min und 60 min Reaktionszeit) werden vom Eigengewicht eines 100 g schweren Stempels zerdrückt. Unbehandelte Celluloseperlen werden durch das Eigengewicht dieses Stempels nur reversibel verformt. Unbehandelte Celluloseperlen (Blindwert) werden erst ab einem Stempel mit einem Eigengewicht von 3 kg irreversibel verformt und ab 5 kg zerdrückt. Die zerdrückten enzymatisch behandelten Celluloseperlen zeigen eine hydrogel-artige Konsistenz und sind weder klebrig noch schmierig.

Ähnlich der enzymatischen Behandlung hat die strukturelle Integrität der Kügelchen auch durch die TEMPO-Oxidation stark abgenommen. So lassen sich diese Kügelchen bereits mit einem 1 kg-Stempel irreversibel mit Rissbildung verformen. Ab einem 2 kg-Stempel zeigt sich eine starke Rissbildung und mit einem 5 kg-Stempel werden die Celluloseperlen bereits vollständig zerdrückt.

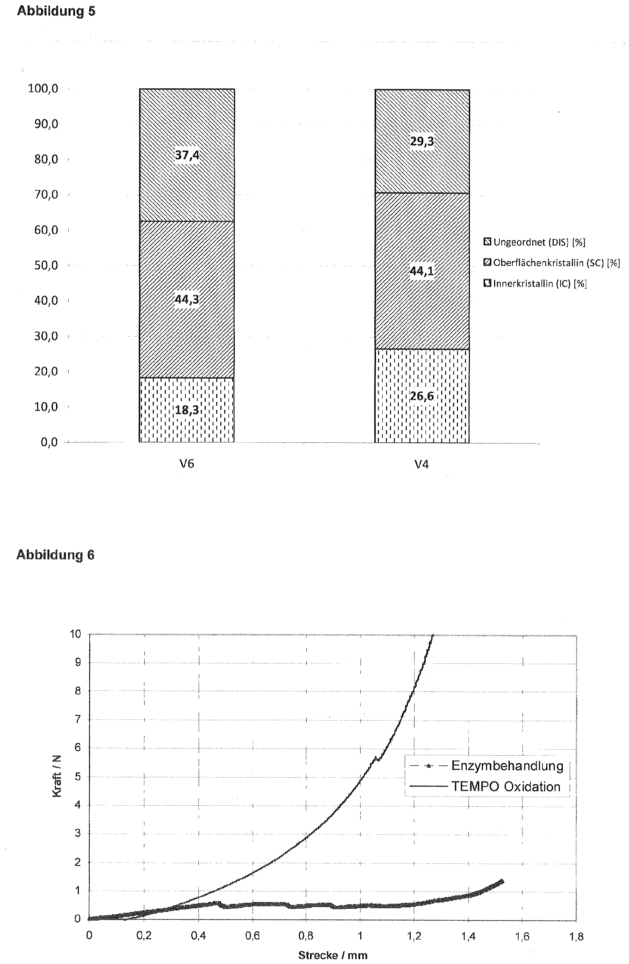

Neben diesen einfachen Versuchen zur Bestimmung der Härte wurden von ausgewählten Proben auch die Mikrohärte mittels eines Härtetestgerätes Shimadzu EZ Test X bestimmt. Durchgeführt wurde die Prüfung mit einem 10 mm-Stempel und die Deformation gegen die aufgebrachte Kraft wurde aufgezeichnet. Abbildung 5 zeigt die Deformationskurven für Enzym behandelte und TEMPO oxidierte Proben.

Bei den enzymatisch behandelten Proben zeigte sich eine drastische Änderung der Morphologie. Neben einer Glättung der Oberfläche kam es auch zu einem Verschwinden der Kern/Hülle-Struktur. Dies zeigt, dass sowohl die dichte Außenhaut als auch das Innere der Celluloseperlen für die Enzyme zugänglich sind und abgebaut werden. Es war kein Unterschied mehr zwischen der Außenhaut und dem Inneren der Celluloseperlen zu erkennen; beide sind Bestandteil eines Hydrogels.

Diese morphologischen Veränderungen bei TEMPO-Oxidation bzw. Enzymbehandlung bestätigen sich auch durch NMR-Untersuchungen (siehe Abbildung 6) wie bereits in Beispiel 5 beschrieben. So nimmt der ungeordnete Celluloseanteil bei der Enzymbehandlung ab und der innerkristalline Anteil steigt dadurch zwangsläufig. Bei der TEMPO-Oxidation nimmt der oberflächenkristalline Anteil stark ab, wodurch der innerkristalline Anteil zunimmt.

### Beispiel 8 - Inkorporation von anorganischen Pigmenten

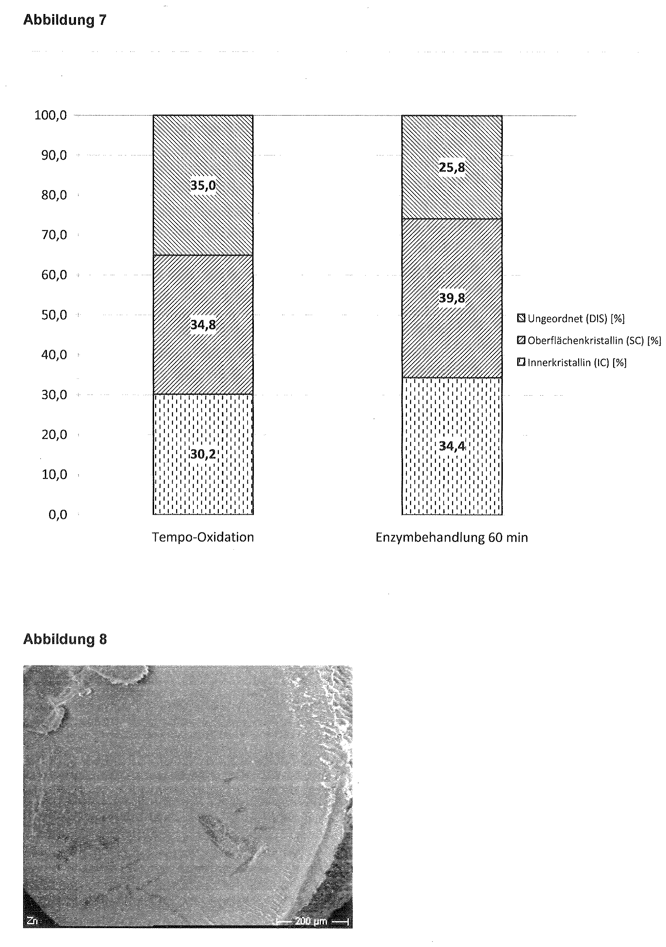

Eine 33 Gew.-% ZnO-Suspension (Type Pharma 4, d₅₀ = 1,2 µm) in 60 Gew.-% wässerigem NMMO wurde zur Herstellung einer Lyocellspinnmasse eingesetzt. Die fertige Spinnmasse bestand aus 12,2 Gew.-% Zellstoff (Typ Bacell), 73,9 Gew.-% NMMO, 11,5 Gew.-% Wasser, 2,4 Gew.-% ZnO sowie Spuren an Stabilisator. Aus dieser Spinnmasse wurden nun Celluloseperlen wie in Beispiel 1, V1, beschrieben hergestellt. Das äußere Erscheinungsbild der Celluloseperlen entsprach jenem der Celluloseperlen ohne ZnO-Zugabe. Der ZnO-Gehalt der Partikel wurde mit 16,7 Gew.-% bestimmt (Veraschung der Kügelchen bei 850°C und gravimetrische Auswertung des Rückstands gegen den Blindwert eines Kügelchens ohne ZnO) und die Verteilung mittels SEM (Hitachi S-4000 Field Emission SEM) mit einem eingebauten EDX (Oxford EDX Detector) gemessen. Abbildung 7 zeigt die sehr gleichmäßige Zn-Verteilung in einem solchen halbierten Tencel-Bead.

## Patentansprüche

1. Dreidimensionaler cellulosischer Formkörper, der eine optisch feststellbare Kern-Mantel-Struktur aufweist, wobei der Mantel eine höhere Dichte und eine niedrigere Kristallinität als der Kern aufweist und der Kern eine schwammähnliche Struktur aufweist, **dadurch gekennzeichnet, dass** der Mantel eine relative Dichte von 65% bis 85% und der Kern eine relative Dichte von 20% bis 60% aufweist - bezogen auf kompakte Cellulose -, die Manteldicke zwischen 50 µm und 200 µm beträgt, das Verhältnis von Manteldicke zu Gesamtdurchmesser des Formkörpers zwischen 1:5 und 1:50 beträgt und das Verhältnis der Halbachsen des Formkörpers 3:1 nicht übersteigt.

2. Formkörper nach Anspruch 1, wobei die nie-getrocknete Variante der Celluloseperlen einen Feuchtegehalt von 25 - 300 Gew.-%, bezogen auf die Cellulosemenge, aufweist.

3. Formkörper nach Anspruch 1, wobei der Formkörper Additivsubstanzen enthält, die während seiner Herstellung inkorporiert wurden und diese Additivsubstanzen ausgewählt sind aus der Gruppe enthaltend ZnO, TiO₂, CaCO₃, Kaolin, Fe₂O₃, Farbpigmente auf Kunststoffbasis, Aktivkohle, Superabsorbermaterialien, Phase-change-Materialien, Flammschutzmittel, Biozide, Chitosan sowie weitere Polymere oder Biopolymere.

4. Verfahren zur Herstellung eines dreidimensionalen cellulosischen Formkörpers mit optisch feststellbarer Kern-Mantel-Struktur, schwammähnlicher Struktur des Kerns und einem Verhältnis der Halbachsen des Formkörpers von nicht mehr als 3:1, **dadurch gekennzeichnet, dass** erfolgende Herstellungsschritte umfasst:
a. Auflösung der Cellulose gemäß einem Lyocellprozess, um eine Lösung mit 10 bis 15 Gew.-% Cellulose zu erhalten;
b. Extrusion der in Schritt a. erhaltenen Celluloselösung ohne Luftspalt direkt in ein Fällbad;
c. Regenerationsprozess, wobei beim Eintritt der Celluloselösung ins Fällbad die Differenz der NMMO-Konzentrationen von Celluloselösung und Fällbad 15 - 78 Gew.-%, bevorzugt 40 - 70 Gew.-%, und die Differenz der Temperatur von Celluloselösung und Fällbad 50 - 120 K, bevorzugt 70 - 120 K, besonders bevorzugt 80 - 120 K betragen sollen;
d. Waschprozess gemäß dem Perkolationsprinzip mit mindestens einem alkalischen Waschschritt, bevorzugt bei pH 9 - 13;
e. optional einem Trocknungsprozess, durch den die Außenhaut der Formkörper nicht abrasiv verletzt wird;
wobei die in Punkt d.) genannte Wäsche bevorzugt mehrstufig und im Gegenstrom erfolgt und mindestens einen alkalischen Schritt enthält.

5. Verfahren gemäß Anspruch 4, wobei der Trocknungsprozess durch NormaldruckTrocknung, Strömungstrocknung, Bandtrocknung, Wirbelschichttrocknung, Gefriertrocknung oder superkritische CO₂-Trocknung erfolgt.

6. Verfahren gemäß Anspruch 4, wobei zwischen Schritt d. und Schritt e. eine Enzymbehandlung stattfindet.

7. Verfahren gemäß Anspruch 6, wobei eines oder mehrere Enzyme, ausgewählt aus der Gruppe enthaltend Exo- und Endo-1,4-b-glucanasen, Glucosidasen und Xylanasen, eingesetzt werden.

8. Verwendung der Formkörper nach Anspruch 1 zur Herstellung eines wirkstoffbeladenen Trägermaterials, wobei der Formkörper mit einer Lösung des Wirkstoffes getränkt und anschließend gewaschen und getrocknet wird.

9. Verwendung der Formkörper nach Anspruch 1 zur Herstellung eines wirkstoffbeladenen Trägermaterials mit Controlled-Release-Eigenschaften, insbesondere für kosmetische und pharmazeutische Anwendungen.

10. Verwendung der Formkörper nach Anspruch 1 als abrasives Material in kosmetischen Produkten wie zum Beispiel Peelings oder Exfoliator-Mischungen, wobei die durchschnittliche Größe der Formkörper 150 - 800 µm, bevorzugt 200 - 800 µm, besonders bevorzugt 300 - 550 µm beträgt.

11. Verwendung der Formkörper nach Anspruch 1 als optische Effektkügelchen in kosmetischen Produkten, bevorzugt Shampoos und Cremes.

12. Verwendung der Formkörper nach Anspruch 1 als Ausgangsmaterial zur Herstellung von sphärischen cellulosischen Pulvern, die Sensoric-Booster-Eigenschaften für kosmetische Produkte in Öl/Wasser-Emulsionen aufweisen, durch Mahlung.

13. Verwendung der Formkörper nach Anspruch 1 als Ausgangsmaterial zur Herstellung von sphärischen cellulosischen Pulvern, welches für Control Release Partikel oder als Kern für Control release Partikel verwendet werden können.

14. Verwendung der Formkörper nach Anspruch 1 als Säulenmaterial in der Chromatographie, insbesondere in der Normalphasen-, Umkehrphasen-, lonenaustausch-, Affinitäts- und Größenausschlußchromatographie.

## Claims

1. A three-dimensional cellulosic molded body having an optically detectable core/shell structure, the shell having a higher density and lower crystallinity than the core and the core having a sponge-like structure, **characterized in that** the shell has a relative density from 65% to 85% and the core has a relative density from 20% to 60% - related to compact cellulose -, shell thickness is between 50 µm and 200 µm, the ratio of the shell thickness to the total diameter of the molded body is between 1:5 and 1:50, and the ratio of the semiaxes of the molded body does not exceed 3:1.

2. The molded body as claimed in claim 1 wherein the never-dried variant of the cellulose beads has a moisture content of 25 - 300% by weight, related to the cellulose quantity.

3. The molded body as claimed in claim 1 wherein the molded body contains additive substances that were incorporated during its production and these additive substances are selected from the group comprising ZnO, TiO₂, CaCO₃, kaolin, Fe₂O₃, plastics-based color pigments, activated carbon, superabsorbent materials, phase change materials, flame retardants, biocides, chitosan, as well as other polymers or biopolymers.

4. A method for the production of a three-dimensional cellulosic molded body having an optically detectable core/shell structure, a core having a sponge-like structure, and a ratio of the semiaxes of the molded body not greater than 3:1, **characterized in that** it comprises the following production steps:
a. dissolution of the cellulose according to a lyocell process in order to obtain a solution having 10 to 15% by weight of cellulose;
b. extrusion of the cellulose solution obtained in step a. without air gap directly into a precipitation bath;
c. a regeneration process wherein, upon entry of the cellulose solution into the precipitation bath, the difference between the NMMO concentrations of the cellulose solution and the precipitation bath is to be 15 - 78% by weight, preferably 40 - 70% by weight, and the difference between the temperatures of the cellulose solution and the precipitation bath is to be 50 - 120 K, preferably 70 - 120 K, more preferably 80 - 120 K;
d. a washing process according to the percolation principle having at least one alkaline washing step, preferably at pH 9 - 13;
e. optionally, a drying process that does not abrasively damage the outer skin of the molded bodies;
the washing process referred to in d.) preferably being conducted in several stages and in countercurrent and containing at least one alkaline step.

5. The method as claimed in claim 4 wherein the drying process is carried out by normal pressure drying, gas stream drying, belt drying, fluidized bed drying, freeze drying, or supercritical CO₂ drying.

6. The method as claimed in claim 4 wherein an enzymatic treatment takes place between step d. and step e.

7. The method as claimed in claim 6 wherein one or several enzymes are used, selected from the group comprising exo- and endo-1,4-b-glucanases, glucosidases, and xylanases.

8. The use of the molded bodies as claimed in claim 1 for the production of an active-agent-loaded carrier material wherein the molded body is soaked with a solution of the active agent and then washed and dried.

9. The use of the molded bodies as claimed in claim 1 for the production of an active-agent-loaded carrier material with controlled release properties, particularly for cosmetic and pharmaceutical applications.

10. The use of the molded bodies as claimed in claim 1 as an abrasive material in cosmetic products such as peelings or exfoliator mixtures, wherein the average size of the molded bodies is 150 - 800 µm, preferably 200 - 800 µm, more preferably 300 - 550 µm.

11. The use of the molded bodies as claimed in claim 1 as optical effect beads in cosmetic products, preferably in shampoos and creams.

12. The use of the molded bodies as claimed in claim 1 as a starting material for the production, by grinding, of spherical cellulosic powders having sensoric booster properties for cosmetic products in oil/water emulsions.

13. The use of the molded bodies as claimed in claim 1 as a starting material for the production of spherical cellulosic powders that can be used for control release particles or as a core for control release particles.

14. The use of the molded bodies as claimed in claim 1 as a column material in chromatography, particularly in normal phase, reversed phase, ion exchange, affinity, and size exclusion chromatography.

## Revendications

1. Corps moulé cellulosique tridimensionnel qui présente une structure noyau-enveloppe visuellement identifiable, l'enveloppe présentant une densité supérieure et une cristallinité inférieure au noyau, et le noyau présentant une structure spongieuse, **caractérisé en ce que** l'enveloppe présente une densité relative de 65 % à 85 % et le noyau une densité relative de 20 % à 60 % - rapportée à de la cellulose compacte -, l'épaisseur de l'enveloppe est comprise entre 50 µm et 200 µm, le rapport entre l'épaisseur de l'enveloppe et le diamètre total du corps moulé est compris entre 1 pour 5 et 1 pour 50, et le rapport des demi-axes du corps moulé ne dépasse pas 3 pour 1.

2. Corps moulé selon la revendication 1, la variante jamais séchée des perles de cellulose présentant une humidité de 25 à 300 % en masse, rapportée à la quantité de cellulose.

3. Corps moulé selon la revendication 1, le corps moulé contenant des substances additives qui ont été incorporées pendant sa fabrication, ces substances additives ayant été choisies dans le groupe comprenant du ZnO, du TiO₂, du CaCO₃, du kaolin, du Fe₂O₃, des pigments colorés à base de matières synthétiques, du charbon actif, des matériaux superabsorbants, des matériaux à changement de phase, des retardateurs de flammes, des biocides, du chitosan ainsi que d'autres polymères et biopolymères.

4. Procédé de fabrication d'un corps moulé cellulosique tridimensionnel avec une structure noyau-enveloppe visuellement identifiable, une structure spongieuse du noyau et un rapport des demi-axes du corps moulé ne dépassant pas 3 pour 1, **caractérisé en ce qu'**il comprend les étapes de fabrication suivantes :
a. dissolution de la cellulose suivant un procédé Lyocell pour obtenir une solution avec 10 à 15 % en masse de cellulose ;
b. extrusion de la solution de cellulose obtenue à l'étape a., sans intervalle d'air directement dans un bain de précipitation ;
c. procédé de régénération dans lequel, à l'entrée de la solution de cellulose dans le bain de précipitation, la différence des concentrations de NMMO de la solution de cellulose et du bain de précipitation doit être comprise entre 15 et 78 % en masse, de préférence entre 40 et 70 % en masse, et la différence de température entre la solution de cellulose et le bain de précipitation doit être comprise entre 50 et 120 K, de préférence entre 70 et 120 K, ou encore mieux entre 80 et 120 K ;
d. procédé de lavage suivant le principe de percolation avec au moins une étape de lavage alcalin, de préférence avec un pH de 9 à 13 ;
e. en option, un procédé de séchage par lequel l'enveloppe du corps moulé n'est pas abîmée par abrasion ;
le lavage cité au point d.) étant réalisé à contre-courant et comportant de préférence plusieurs phases et au moins une étape alcaline.

5. Procédé selon la revendication 4, le procédé de séchage étant effectué par séchage à pression normale, séchage par soufflage de gaz, séchage par bande, séchage par lit fluidisé, lyophilisation ou séchage au CO₂ supercritique.

6. Procédé selon la revendication 4, un traitement enzymatique ayant lieu entre l'étape d. et l'étape e.

7. Procédé selon la revendication 6 dans lequel sont utilisées une ou plusieurs enzymes sélectionnées dans le groupe comprenant les exo- et endo-1,4-b-glucanases, les glucosidases et les xylanases.

8. Utilisation des corps moulés selon la revendication 1 pour la fabrication d'un support chargé de substances actives, le corps moulé étant imprégné d'une solution de la substance active, puis lavé et séché.

9. Utilisation des corps moulés selon la revendication 1 pour la fabrication d'un support chargé de substances actives ayant des propriétés de libération contrôlée (« controlled release »), en particulier pour les applications cosmétiques et pharmaceutiques.

10. Utilisation des corps moulés selon la revendication 1 comme matière abrasive dans des produits cosmétiques comme les peelings ou mélanges exfoliants, la taille moyenne des corps moulés étant comprise entre 150 et 800 µm, de préférence entre 200 et 800 µm, ou encore mieux entre 300 et 550 µm.

11. Utilisation des corps moulés selon la revendication 1 comme petites billes à effet optique dans des produits cosmétiques, de préférence des shampoings et des crèmes.

12. Utilisation des corps moulés selon la revendication 1 comme matière de base pour la fabrication par mouture de poudres cellulosiques sphériques présentant des propriétés de stimulation sensorielle (« sensoric booster ») pour les produits cosmétiques dans des émulsions huile-eau.

13. Utilisation des corps moulés selon la revendication 1 comme matière de base pour la fabrication de poudres cellulosiques sphériques pouvant être utilisées pour des particules à libération contrôlée ou comme noyau pour des particules à libération contrôlée.

14. Utilisation des corps moulés selon la revendication 1 comme matière pour colonnes en chromatographie, en particulier en chromatographie en phase normale, en phase inverse, à échange d'ions, d'affinité et à exclusion stérique.
